# EUROPÄISCHE PATENTSCHRIFT

(11) **EP 1 980 285 B1**
(45) Veröffentlichungstag und Bekanntmachung des Hinweises auf die Patenterteilung: **18.11.2009**
(21) Anmeldenummer: 07007418.2
(22) Anmeldetag: 11.04.2007
(51) Int. Cl.: A61M 11/00

(54) **Aerosoltherapievorrichtung**
Aerosol therapy device
Dispositif de thérapie par aérosol

(43) Veröffentlichungstag der Anmeldung: 15.10.2008
(73) Patentinhaber: PARI GmbH Spezialisten für effektive Inhalation, 82319 Starnberg (DE)
(72) Erfinder: Schuschnig, Uwe, 81371 München (DE); Luber, Martin, 81545 München (DE); Achtzehner, Wolfgang, 82239 Alling (DE)
(74) Vertreter: HOFFMANN EITLE

(56) Entgegenhaltungen:
- EP-A1- 0 507 707
- DE-B3- 10 239 321
- US-A1- 2005 034 719

## Beschreibung

Die Erfindung betrifft eine Aerosoltherapievorrichtung, bei der über ein Nasenstück ein in einem Vernebler erzeugtes Aerosol in Form einer Aerosolströmung den Nasenhöhlen eines Patienten zugeführt wird.

In diesem Zusammenhang ist aus "Eindringvermögen von Aerosolen in Nebenräume", H.Kauff, Archiv klin. exper. Ohren-, Nasen- und Kehlkopfheilk. 190, 95-108 (1968) bekannt, dass Druckschwankungen und Vibrationen der Anlass sein können, dass Aerosol in die Nasennebenhöhlen eindringt, die ansonsten von der Aerosolströmung durch die Nasenhöhlen nicht aktiv durchströmt werden. Ein Anwendungsbeispiel dieser Erkenntnisse ist aus der EP 0 507 707 A1 bekannt. Danach werden einer Aerosolströmung Druckschwankungen überlagert, die bewirken sollen, dass Aerosolpartikel/-tröpfchen der Aerosolströmung die Ostien passieren und in die Nasennebenhöhlen gelangen. Auf diese Weise können auch die Nasennebenhöhlen, obwohl von der Aerosolströmung nicht unmittelbar durchströmt, von einem in Aerosolform dargebotenen Medikament erreicht und therapiert werden. Wie auch bei anderen Aerosoltherapien wird angestrebt, das Medikament in ausreichender Menge an den gewünschten Stellen zu deponieren, wozu im Fall der Nasennebenhöhlen eine ausreichende Menge des Aerosols der Aerosolströmung die Ostien passieren und in die Nasennebenhöhlen eindringen muss.

Experimentelle Untersuchungen an verschiedenen Modellen der menschlichen Nase haben gezeigt, dass die Deposition in den Nasennebenhöhlen bei Anwendung der bekannten Medikament in ausreichender Menge an den gewünschten Stellen zu deponieren, wozu im Fall der Nasennebenhöhlen eine ausreichende Menge des Aerosols der Aerosolströmung die Ostien passieren und in die Nasennebenhöhlen eindringen muss.

Experimentelle Untersuchungen an verschiedenen Modellen der menschlichen Nase haben gezeigt, dass die Deposition in den Nasennebenhöhlen bei Anwendung der bekannten Aerosoltherapievorrichtungen geringer ist als erwartet und gewünscht. Auch die Öffnungsgröße der Ostien, die krankheitsbedingt oftmals sehr klein ist, hat einen starken Einfluss auf die Deposition.

Aus der DE 102 39 321 B3 ist eine Aerosoltherapievorrichtung der oben beschriebenen Art bekannt, mit einem Vernebler umfassend einen Aerosolgenerator, dem Druckluft für die Erzeugung einer Aerosolströmung zugeführt wird, und einen Anschluss für die Zuführung von Druckschwankungen, die der Aerosolströmung überlagert werden, und einem Nasenstück für die Zuführung des Aerosols in einen der beiden Nasenflügel bzw. -öffnungen eines Benutzers, das mit dem Vernebler verbunden ist. Ferner ist eine Strömungswiderstandseinrichtung vorgesehen, mit der der Strömungswiderstand an der anderen der beiden Nasenöffnungen des Benutzers genau definiert wird. Nur durch den Strömungswiderstand an der anderen Nasenöffnung führen die überlagerten Druckschwankungen dazu, dass Aerosol aus der Aerosolströmung auch in die Nasennebenhöhlen gelangt und dort eine Deposition des Aerosols stattfindet.

Die in der DE 102 39 321 B3 beschriebene Zuführung der Druckgasströmung und der Druckschwankungen erfordert aber eine besondere Gestaltung des Verneblers, so dass sich nicht jeder Vernebler für diese Anwendung eignet.

Um diese Problematik zu beheben, beschreibt die nachveröffentlichte DE 10 2006 001 113 B eine Aerosoltherapievorrichtung umfassend einen Vernebler zum Erzeugen eines Aerosols und Ausbilden einer Aerosolströmung, ein erstes Nasenstück zum Einbringen der Aerosolströmung in eine der beiden Nasenöffnungen eines Verwenders, eine Druckschwankungsquelle zum Erzeugen einer Druckschwankung und ein zweites Nasenstück zum Einbringen der Druckschwankung in die andere der beiden Nasenöffnungen des Verwenders, um die Druckschwankung und die Aerosolströmung zu überlagern.

Insbesondere im Rahmen einer Antifungal- oder Antibiotikatherapie der Nasennebenhöhlen ist eine der regulatorischen Anforderungen an eine Aerosoltherapievorrichtung die Einschränkung bzw. Vermeidung der Wirkstofffremddeposition im Rachenraum und in der Lunge.

Um diese Anforderung zu erfüllen, muss mit der Aerosoltherapievorrichtung gewährleistet sein, dass nur eine Aerosolapplikation in die Nase erfolgt, wenn das Gaumensegel des Anwenders geschlossen ist.

Das der vorliegenden Erfindung zu Grunde liegende Problem beruht darauf eine Aerosoltherapievorrichtung bereitzustellen, bei der auf einfachste Art und Weise auf den Schließungsgrad des Gaumensegels des Verwenders geschlossen werden kann.

Der vorliegenden Erfindung liegt der Gedanke zu Grunde, dass die Druckschwankung, die in eine Nasenöffnung eingebracht wird, bei geschlossenem Gaumensegel durch die gesamte Nasenhöhle übertragen wird und sich mit geringer Dämpfung bis zu dem an der anderen Nasenöffnung befindlichen Vernebler fortpflanzt. Ist das Gaumensegel geöffnet oder nur teilweise geschlossen, so wird durch die Voluminavergrößerung (Rachenraum, Lunge etc.) und durch die zusätzlich erhöhte Weichteildämpfung das Signal der Druckschwankung nur stark gedämpft ankommen bzw. ausgelöscht. D. h. es lässt sich an der Nasenöffnung, in die das Aerosol eingebracht wird anhand der Höhe (Amplitude) des ankommenden Druckschwankungssignals bestimmen, ob das Gaumensegel zu einem entsprechenden Grad geschlossen ist oder nicht. Voraussetzung ist hierfür allerdings eine dichte Anbindung der zwei Nasenstücke an die Nase, was indirekt durch die vorliegende Erfindung (Auswerteeinrichtung) mit beurteilt werden kann.

Die obige Aufgabe wird durch eine Aerosoltherapievorrichtung der vorliegenden Erfindung, wie sie im Patentanspruch 1 definiert ist, gelöst. Vorteilhafte Ausbildungen sind in den Unteransprüchen genannt.

Es sei angemerkt, dass das erste und zweite Nasenstück integral oder aber separat ausgebildet sein können.

Vorzugsweise umfasst der Vernebler zum Erzeugen des Aerosols eine Membran und einen mit der Membran in Wirkverbindung stehenden elektromechanischen Wandler, insbesondere ein piezoelektrisches Element, wobei der erste Drucksensor, der die an dem ersten Nasenstück ankommenden Druckschwankungen erfasst durch diese Elemente gebildet ist. Dadurch kann die Anzahl der Teile stark reduziert und daher die Ausgestaltung der gesamten Aerosoltherapievorrichtung vereinfacht werden. Das bringt sowohl hinsichtlich der Montage als auch der Kosten Vorteile. Bei dieser Ausgestaltung trifft die eingebracht Druckschwankung über das erste Nasenstück auf die Membran und versetzt die Membran in Schwingung. Diese Schwingung führt wiederum zu einer Deformation des elektromechanischen Wandlers. Die Deformation erzeugt ein elektrisches Ausgangssignal, über welches die Druckschwankung erfasst und ausgewertet werden kann, um auf den Schließungsgrad des Gaumensegels zu schließen. Bezüglich der Ausgestaltung einer solchen Membran als Sensor wird auf die EP 1 304 131 A1 verwiesen, in der die Membran als "Atemsensor" eingesetzt wird. Alternativ ist es jedoch auch denkbar einen separaten Drucksensor einzusetzen statt die Membran des Verneblers als ersten Drucksensor zu verwenden.

Dies ist insbesondere auch bei einem Düsenvernebler vorzusehen, der keine solche Membran aufweist.

Vorteilhafterweise regelt die Auswerteeinrichtung in Abhängigkeit des Schließungsgrades die Aerosoltherapievorrichtung. D. h. je nach ankommender Signalhöhe bzw. Signalverschiebung durch den Schließungsgrad des Gaumensegels soll die Aerosolproduktion und/oder die Aerosolströmung ausgelöst/ausgebildet bzw. beendet werden. Dadurch wird gewährleistet, dass nur bei geschlossenem Gaumensegel Wirkstoff durch die Nasenhöhle strömt und eine Wirkstoffdepositionen in dem Rachenraum bzw. Lunge ist ausreichend stark minimiert bzw. vermieden.

Darüber hinaus ist es von Vorteil, wenn die Auswerteeinrichtung in Abhängigkeit des Schließungsgrades ein akustisches und/oder optisches Signal ausgibt, um den Schließungsgrad anzugeben. Dadurch wird dem Anwender die Koordination beim Schließen des Gaumensegels erleichtert. Auch kann eine Art Trainingsmodus zur Verfügung gestellt werden mit dem der Anwender das Schließen des Gaumensegels trainieren kann.

Vorzugweise ist das erfasste Signal der Druckschwankung ihre Frequenz und/oder ihr Druck. Die Frequenz liegt dabei in einem Bereich von ca. 30-50 Hz und ist unabhängig von den physischen Gegebenheiten des jeweiligen Verwenders. In Abhängigkeit von den entsprechenden Volumina des Verwenders (Nasenhöhlen) ergibt sich lediglich eine Phasenverschiebung der Frequenz. Alternativ ist es jedoch auch möglich den Druck zu erfassen. Um entsprechend auf den Schließungsgrad zu schließen, wird dabei die Frequenz oder der Druck über die Zeit gemessen und entsprechend mit vorgegebenen Werten verglichen.

Als insbesondere vorteilhaft hat sich die Verwendung einer sinusförmigen Druckschwankung erwiesen.

Vorteilhafterweise weist die Aerosoltherapievorrichtung einen zweiten Drucksensor auf, der das Signal der Druckschwankung erfasst, die über das zweite Nasenstück in die andere der beiden Nasenöffnungen des Verwenders eingebracht wird, wobei die Auswerteeinrichtung die Signale von dem ersten und zweiten Drucksensor vergleicht, um auf den Schließungsgrad des Gaumensegels des Verwenders zu schließen zu können.

Weitere Vorteile und Merkmale der vorliegenden Erfindung sind aus der folgenden Beschreibung einer beispielhaften Ausführungsform ersichtlich, die unter Bezugnahme auf die begleitenden Zeichnungen erfolgt.

In den Zeichnungen zeigt:
Fig. 1 eine schematische Darstellung einer Aerosoltherapievorrichtung gemäß der vorliegenden Erfindung.
Fig. 2 eine beispielhafte Darstellung der eingebrachten Druckschwankung sowie des an dem ersten Nasenstück ankommenden Signals bei entsprechend vollständig geöffnetem Gaumensegel, teilweise geöffnetem Gaumensegel und geschlossenem Gaumensegel.

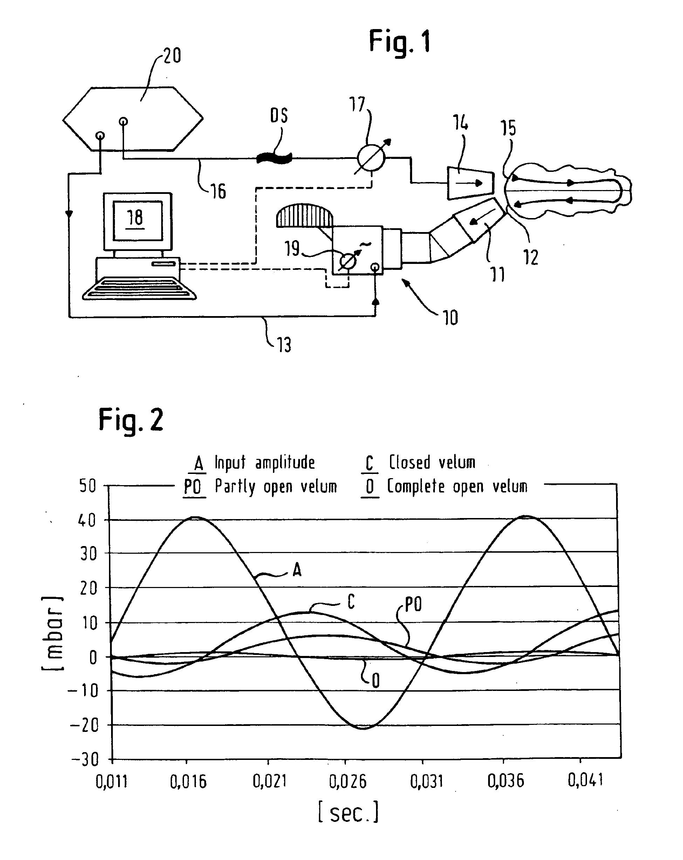

Fig. 1 zeigt ein Beispiel einer Aerosoltherapievorrichtung gemäß einer Ausführungsform der vorliegenden Erfindung mit einem Vernebler 10. Der Vernebler 10 weist zum Erzeugen eines Aerosols eine Membran und eine mit dieser in Wirkverbindung stehenden elektromechanischen Wandler - hier ein piezoelektrisches Element - auf. Über den elektromechanischen Wandler wird die Membran in Schwingung versetzt, so dass der bevorzugt in flüssiger Form vorliegende Wirkstoff durch die Membran hindurch in eine (nicht dargestellte) Verneblungskammer vernebelt wird. Diesen Vorgang bezeichnet man auch als Aerosolproduktion. Bezüglich der an sich bekannten Ausgestaltung der Membran sowie der späteren Nutzung als Sensor wird der Fachmann für weitergehende Informationen auf die EP 1 304 131 A1 verwiesen.

Mit der Verneblungskammer in Fluidverbindung steht ein erstes 11 Nasenstück zum dichten Anbinden an eine der beiden Nasenöffnungen 12 eines Verwenders. Die Aerosolströmung wird in der dargestellten Ausführungsform über eine Verbindung des Verneblers 10 mit einer Versorgungseinheit 20 ausgebildet. Dazu sind die Versorgungseinheit 20 und der Vernebler 10 über eine Druckluftleitung 13 miteinander verbunden, über die Druckluft in die Verneblerungskammer eingebracht, um die Aerosolströmung auszubilden, die über das erste Nasenstück 11 in die Nasenöffnung 12 eingebracht wird.

Ferner umfasst die Aerosoltherapievorrichtung der vorliegenden Erfindung ein zweites Nasenstück 14, das an die andere der beiden Nasenöffnungen 15 dicht anzubinden ist. Das zweite Nasenstück 14 ist über eine Druckschwankungsleitung 16 mit der Versorgungseinheit 20 verbunden. Die Versorgungseinheit 20 enthält eine (nicht dargestellte) Druckschwankungsquelle über die eine sinusförmige Druckschwankung erzeugt wird. Die Druckschwankung wird über die Druckschwankungsleitung 16 und das zweite Nasenstück 14 in die Nasenöffnung 15 eingebracht.

Im Betrieb überlagen sich daher die Aerosolströmung und die Druckschwankung in der Nasenhöhle, um eine bevorzugte Deposition des Wirkstoffes in den Nasennebenhöhlen zu gewährleisten.

Ferner ist in der Druckschwankungsleitung 16 ein Drucksensor 17 (zweiter Drucksensor) vorgesehen, der die einzubringende Druckschwankung - die von der Quelle erzeugte Druckschwankung - erfasst. Der Drucksensor 17 ist mit einer Auswerteeinheit 18 verbunden. Die Membran des Verneblers 10 steht ebenfalls mit der Auswerteeinrichtung 18 in Verbindung. Wie es in der EP 1 304 131 A1 beschrieben ist, kann diese Membran als Drucksensor (erster Drucksensor) eingesetzt werden. Mit anderen Worten versetzt die an dem Nasenstück 11 ankommende Druckschwankung, die in die Verneblerungskammer gelangt und damit auf die Membran trifft, die Membran in Schwingung, wodurch das piezoelektrische Element verformt wird und ein elektrisches Signal ausgibt. Dieses ausgegebene elektrische Signal kann von der Auswerteeinheit 18 genutzt werden, um die Druckschwankung zu messen. Alternativ und wie es in Fig. 1 dargestellt ist, kann auch ein separater Drucksensor 19 (erster Drucksensor) in der Verneblungskammer oder dessen Wandungen bzw. dem Nasenstück 11 vorgesehen sein, um die Druckschwankung zu messen.

Bezüglich der Ausgestaltung der Nasenstücke sowie der Verbindungmit dem Vernebler wird auf die DE 102 39 321 B3 bzw. die nachveröffentlichte DE 10 2006 001 113 B verwiesen. Gleiches gilt auch für die Einrichtungen zum Ausbilden der Druckschwankung, d. h. die Druckschwankungsquelle.

Im Folgenden wird unter Bezugnahme auf die Fig. 1 und 2 der Betrieb der erfindungsgemäßen Aerosoltherapievorrichtung, wie sie zuvor beschrieben wurde, erläutert.

Im Betrieb erzeugt die Druckschwankungsquelle der Versorgungseinrichtung 20 eine Druckschwankung, die eine sinusförmige, treppenförmige, gepulste oder eine andere Form haben kann. Die hier sinusförmige Durckschwankung wird über die Versorgungsleitung 16 und das zweite Nasenstück 14 in die Nasenöffnung 15 des Verwenders eingebracht. Die Auswerteeinrichtung 18 misst über den Drucksensor 17 die eingebrachte Druckschwankung, wie sie in Fig. 2 mit A (Inputamplitude) gekennzeichnet ist. Die eingebrachte Druckschwankung A (Inputamplitude) pflanzt sich von der Nasenöffnung 15, wie es in Fig. 1 mit DS gekennzeichnet ist, in der Nasenhöhle fort und tritt aus der Nasenöffnung 12 aus. Von dort strömt sie durch das erste Nasenstück 11 und wird im dargestellten Beispiel durch den Drucksensor 19 erfasst. Das erfasste Signal wird an die Auswerteeinheit 18 ausgegeben und kann dort mit dem Wert des Drucksensors 17 verglichen werden.

Entspricht das durch den ersten Drucksensor 19 erfasste Signal der Druckschwankung bei geschlossenem Gaumensegel, wie es in Fig. 2 mit C (closed velum) gekennzeichnet ist, so wird bei kontinuierlicher Aerosolproduktion durch Zuführen von Druckluft über die Druckluftleitung 13 in die Verneblungskammer die Aerosolströmung ausgebildet bzw. ausgelöst. Alternativ kann bei kontinuierlicher Zufuhr von Druckluft über die Druckluftleitung 13, d. h. kontinuierlich bestehender Strömung, zu diesem Zeitpunkt die Aerosolproduktion ausgelöst werden, d. h. es wird über die Membran Aerosol erzeugt. Bei entsprechend durch die Bezugsziffern PO (partly open velum) bzw. O (complete open velum) gekennzeichneten Druckschwankungen, die einen teilweise geöffnetes bzw. geöffneten Gaumensegel entsprechen, erfolgt keine derartige Regelung. Aus Fig. 2 ist ferner ersichtlich, dass die Druckschwankungskurve über die Zeit immer flacher wird, d. h. die Dämpfung mit zunehmendem Öffnungsgrad des Gaumensegels stark zunimmt.

Darüber hinaus kann bei der erfindungsgemäßen Vorrichtung vorgesehen sein, dass die einzelnen Schließungsgrade O (complete open velum), PO (partly open velum) und C (closed velum) durch entsprechend verschiedenfarbige Leuchtmittel angezeigt werden oder aber auf einer Skala bei der mit zunehmendem Schließungsgrad ein Leuchtmittel zugeschaltet wird. Auch ist die Ausgabe eines akustischen Signals ggf. in Sprache (Gaumensegel geschlossen) denkbar. Dadurch wird es dem Verwender gestattet das Schließen des Gaumensegels mit einer entsprechenden Rückmeldung durch das System zu trainieren.

Wie es weiter aus Fig. 2 ersichtlich ist, handelt es sich bei der eingebrachten Druckschwankung bei der dargestellten Ausführungsform um eine sinusförmige Druckschwankung, d. h. die Druckschwankung hat die Form einer Sinuskurve.

Wie es aus der obigen Beschreibung ersichtlich wird, beruht die vorliegend Erfindung einerseits darauf das Aerosol in eine der beiden Nasenöffnungen und die Druckschwankung in die andere der beiden Nasenöffnungen einzubringen und die entsprechend auf der Seite, an der das Aerosol eingebracht wird, ankommende Druckschwankung zu messen, um dadurch auf den Schließungsgrad des Gaumensegels zu schließen. Obwohl die vorliegende Erfindung zuvor unter Bezugnahme auf eine spezielle Ausführungsform beschrieben wurde, versteht sich, dass die Erfindung ausschließlich durch die folgenden Patentansprüche definiert und beschränkt ist.

## Patentansprüche

1. Aerosoltherapievorrichtung umfassend:
einen Vernebler (10) zum Erzeugen eines Aerosols und Ausbilden einer Aerosolströmung,
ein erstes Nasenstück (11) zum Einbringen der Aerosolströmung in eine der beiden Nasenöffnungen (12) eines Verwenders,
eine Druckschwankungsquelle zum Erzeugen einer Druckschwankung (DS), und
ein zweites Nasenstück (14) zum Einbringen der Druckschwankung in die andere der beiden Nasenöffnungen (15) des Verwenders, um die Druckschwankung und die Aerosolströmung zu überlagern,
eine Sensorik mit einem ersten Drucksensor (19) zum Erfassen des Signals der Druckschwankung, das an dem ersten Nasenstück (11) ankommt, und
eine Auswerteeinrichtung (18), die über das Signal auf den Schließungsgrad des Gaumensegels des Verwenders schließt.

2. Aerosoltherapievorrichtung nach Anspruch 1, bei der der Vernebler (10) zum Erzeugen des Aerosols eine Membran und einen mit dieser in Wirkverbindung stehenden elektromechanischen Wandler, insbesondere ein piezoelektrisches Element, umfasst und bei der der erste Drucksensor (19) durch diese Elemente gebildet ist.

3. Aerosoltherapievorrichtung nach einem der vorstehenden Ansprüche, bei der die Auswerteeinrichtung (18) in Abhängigkeit des Schließungsgrads die Erzeugen des Aerosols und/oder die Ausbildung der Aerosolströmung auslöst oder beendet.

4. Aerosoltherapievorrichtung nach einem der vorstehenden Ansprüche, bei der die Auswerteeinrichtung in Abhängigkeit des Schließungsgrads ein akustisches und/oder optisches Signal ausgibt, um den Schließungsgrad anzuzeigen.

5. Aerosoltherapievorrichtung nach einem der vorstehenden Ansprüche, bei der das erfasste Signal der Druckschwankung ihre Frequenz und/oder ihr Druck ist.

6. Aerosoltherapievorrichtung nach einem der vorstehenden Ansprüche, bei der die Sensorik ferner einen zweiten Drucksensor (17) umfasst, der das Signal der Druckschwankung (DS) erfasst, die über das zweite Nasenstück (14) in die andere der beiden Nasenöffnungen (15) des Verwenders eingebracht wird, wobei die Auswerteeinrichtung (18) die Signale von dem ersten (19) und zweiten (17) Drucksensor vergleicht, um auf den Schließungsgrad des Gaumensegels des Verwenders zu schließen.

7. Aerosoltherapievorrichtung nach einem der vorstehenden Ansprüche, bei der die Druckschwankung eine regelmäßige, insbesondere sinusförmige, Druckschwankung ist.

## Claims

1. Aerosol treatment device comprising:
a nebuliser (10) for generating an aerosol and forming an aerosol stream,
a first nose piece (11) for introducing the aerosol stream into one of the two nostrils (12) of a user,
a pressure-variation source for generating a pressure variation (DS), and
a second nose piece (14) for introducing the pressure variation into the other of the two nostrils (15) of the user in order to superimpose the pressure variation and the aerosol stream,
a sensor system having a first pressure sensor (19) for detecting the signal of the pressure variation which arrives at the first nose piece (11), and
an evaluating device (18) which concludes the degree of closure of the velum of the user via the signal.

2. Aerosol treatment device according to claim 1, in which the nebuliser (10) for generating the aerosol comprises a membrane and an electromechanical transducer in operative connection with the latter, in particular a piezoelectric element, and in which the first pressure sensor (19) is formed by these elements.

3. Aerosol treatment device according to one of the above claims, in which the evaluating device (18) triggers or ends generation of the aerosol and/or the formation of the aerosol stream as a function of the degree of closure.

4. Aerosol treatment device according to one of the above claims, in which the evaluating device emits an acoustic and/or optical signal as a function of the degree of closure to indicate the degree of closure.

5. Aerosol treatment device according to one of the above claims, in which the detected signal of the pressure variation is its frequency and/or its pressure.

6. Aerosol treatment device according to one of the above claims, in which the sensor system also comprises a second pressure sensor (17) which detects the signal of the pressure variation (DS) which is introduced into the other of the two nostrils (15) of the user via the second nose piece (14), wherein the evaluating device (18) compares the signals from the first (19) and second (17) pressure sensor to conclude the degree of closure of the velum of the user.

7. Aerosol treatment device according to one of the above claims, in which the pressure variation is a regular, in particular sinusoidal, pressure variation.

## Revendications

1. Dispositif de thérapie par aérosol, comprenant :
un nébuliseur (10), pour produire un aérosol et former un écoulement d'aérosol,
une première pièce de nez (11) pour introduire l'écoulement aérosol dans l'une des deux ouvertures nasales (17) d'un utilisateur,
une source de fluctuation de pression, pour produire une fluctuation de pression (DS), et
une deuxième pièce de nez (14) pour introduire la fluctuation de pression dans l'autre des deux ouvertures nasales (15) de l'utilisateur, pour superposer la fluctuation de pression et l'écoulement aérosol,
une sensorique, munie d'un premier capteur de pression (19) pour détecter le signal de la fluctuation de pression arrivant à la première pièce de nez (11), et
un dispositif d'évaluation (18), déterminant, par l'intermédiaire du signal, le degré de fermeture du voile de palais de l'utilisateur.

2. Dispositif de thérapie par aérosol selon la revendication 1, dans lequel le nébuliseur (10) pour produire l'aérosol comprend une membrane et un convertisseur électromécanique, en particulier un élément piézoélectrique, placé en liaison fonctionnelle avec celle-ci, et pour lequel le premier capteur de pression (19) est formé par ces éléments.

3. Dispositif de thérapie par aérosol selon l'une des revendications précédentes, dans lequel le dispositif d'évaluation (18) déclenche ou achève la génération de l'aérosol et/ou la réalisation de l'écoulement d'aérosol, en fonction du degré de fermeture.

4. Dispositif de thérapie par aérosol selon l'une des revendications précédentes, dans lequel, en fonction du degré de fermeture, le dispositif d'évaluation émet un signal acoustique et/ou optique, pour afficher le degré de fermeture.

5. Dispositif de thérapie par aérosol selon l'une des revendications précédentes, dans lequel le signal détecté de la fluctuation de pression est sa fréquence et/ou sa pression.

6. Dispositif de thérapie par aérosol selon l'une des revendications précédentes, dans lequel la sensorique comprend en outre un deuxième capteur de pression (17), détectant le signal de la fluctuation de pression (DS), introduite, par la deuxième pièce de nez (14), dans l'autre des deux ouvertures nasales (15) de l'utilisateur, le dispositif d'évaluation (18) comparant les signaux du premier (19) et du deuxième (17) capteur de pression, pour déterminer le degré de fermeture du voile de palais de l'utilisateur.

7. Dispositif de thérapie par aérosol selon l'une des revendications précédentes, dans lequel la fluctuation de pression est une fluctuation de pression régulière, en particulier sinusoïdale.
